(19) **Europäisches Patentamt**
**European Patent Office**
**Office européen des brevets**

(11) **EP 2 218 793 A1**

(12) **EUROPEAN PATENT APPLICATION**

(43) Date of publication:
**18.08.2010 Bulletin 2010/33**

(51) Int Cl.:
**C12Q 1/68** (2006.01)

(21) Application number: **10153536.7**

(22) Date of filing: **12.02.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **13.02.2009 CH 2322009**

(71) Applicant: **Alphagenics International SA**
**1052 Le Mont (CH)**

(72) Inventor: **Morandi, Luca**
**Bologna (IT)**

(74) Representative: **Grosfillier, Philippe**
**Andre Roland S.A.**
**Avenue Tissot 15**
**P.O. Box 1255**
**1001 Lausanne (CH)**

(54) **Detection of MGMT methylation in tumors**

(57) A method for quantifying the methylated allele of 06-methylguanine-DNA methyltransferase (MGMT) promoter DNA in cancer cells, comprising: assaying for quantifying the methylated allele of MGMT promoter DNA in formalin fixed paraffin embedded (FFPE) by methylation sensitive quantitative PCR.

EP 2 218 793 A1

**Description**

**FIELD OF THE INVENTION**

This invention is related to a method for the detection of MGMT methylation in tumors, especially gliomas, using DNA isolated from FFPE or fresh/frozen tissue samples. This method is based on methylation sensitive quantitative real time PCR assay (MS-qPCR) which permits high throughput quantification of the methylation status of the promoter of MGMT gene in an accurate, very sensitive and cost-effective manner. High specificity was achieved recognizing methylated and unmethylated CpGs by 3'-locked nucleic acid (LNA) primers and molecular beacon probes. The promoter methylation status of SNURF was selected as a reference locus. SNURF belongs to the 15q imprinted center mapped on 15q12. The maternal allele is usually methylated, while the paternal one is unmethylated. In theory in a homogeneous population of cells of the same individual the methylated maternal alleles should be balanced with the unmethylated paternal alleles. This feature allows an easy and precise calculation of the ratio between the methylated and unmethylated alleles of MGMT.

**DESCRIPTION OF THE RELATED ART**

Detection of cancer at an early stage is critical for successful treatment and increasing survivability. Cancer arises due to the accumulation of DNA alterations that results in cells to uncontrollably proliferate. The most common DNA alteration (>95%) is epigenomic-caused methylation in the promoter of some specific genes such as tumor suppression genes. Methylation of the specific genes is demonstrated to be early and stable molecular fingerprints of pre-cancer and cancer cells. Methylation of CpG islands involves the course in which DNA methyltransferases (Dnmts) transfer a methyl group from S-adenosyl-L-methionine to the fifth carbon position of the cytosines. It was well demonstrated that methylation patterns of DNA from cancer cells are significantly different from those of normal cells. More than 6000 published research articles described the relationship between cancer and gene methylation, which established a solid basis for gene methylation-characterized cancerous change. There have been many methods for the detection of DNA methylation. The most widely used method among these is methylation-specific PCR (MS-PCR). This assay, through chemical modification of DNA, selectively amplify methylated sequences with primers specific for methylation (Herman et al., Proc. Natl. Acad. Sci. USA 93: 9821-9826 (1996). After PCR a gel-based detection is processed. MS-PCR was recently improved by using real-time probe system. These improved methods such as MethyLight, Q-MSP, and HM-MethyLight showed to be more sensitive, specific, quantitative and high throughput than original MS-PCR (Eads et al., Cancer Res, 59: 2302-2306, 1999; Cottrell et al., Nucleic Acid Res, 32: e10, 2004). Epigenetic silencing of the *MGMT* gene by promoter methylation is associated with loss of MGMT expression[4-6], diminished DNA-repair activity and longer overall survival in patients with glioblastoma (GBM) who, in addition to radiotherapy, received alkylating chemotherapy with carmustine or temozolomide[7-9]. The *MGMT* gene is located on chromosome 10q26 and encodes a DNA-repair protein that removes alkyl groups from the $O^6$ position of guanine, an important site of DNA alkylation. The restoration of the DNA consumes the MGMT protein, which the cell must replenish. Left unrepaired, chemotherapy-induced lesions, especially 06-methylguanine, trigger cytotoxicity and apoptosis[9-10]. High levels of MGMT activity in cancer cells create a resistant phenotype by blunting the therapeutic effect of alkylating agents and may be an important determinant of treatment failure[9-15]. Patients with glioblastoma containing a methylated *MGMT* promoter benefited from temozolomide, whereas those who did not have a methylated *MGMT* promoter did not have such a benefit[16-17]. Given the key roles of cytosine methylation, there has been a huge interest in the development of procedures for DNA methylation analyses [2,3,6,18-27]. The most used method to detect MGMT promoter methylation status is nested Methylation sensitive PCR described by Palmisano et A1.[34]. More recently, Vlassenbroeck I et A1[28] described a Real Time by SYBRGreen method to detect MGMT methylation status. Using this technique, the copy number of the methylated MGMT promoter, normalized to the beta-actin gene, provides a quantitative test result. Woidacz TK et Al. showed that MGMT methylation could be detected at levels as low as 0.1%. by high resolution melting analysis[29].

**BRIEF SUMMARY OF THE INVENTION**

The present invention is related to a novel method based on methylation sensitive quantitative real time PCR assay (MS-qPCR) which permits high throughput quantification of the methylation status of the promoter of MGMT gene in an accurate, very sensitive and cost-effective manner. High specificity was achieved recognizing methylated and unmethylated CpGs by 3'-locked nucleic acid (LNA) primers and molecular beacon probes. The promoter methylation status of SNURF was selected as a reference locus. SNURF belongs to the 15q imprinted center mapped on 15q12. The maternal allele is usually methylated, while the paternal one is unmethylated. In theory in a homogeneous population of cells of the same individual the methylated maternal alleles should be balanced with the unmethylated paternal alleles. This feature allows an easy and precise calculation of the ratio between the methylated and unmethylated alleles of MGMT. By this protocol a sensitivity of 0.1% of methylated alleles in a background of 1.5 µg bisulfite treated DNA was detected and confirmed by the 24 replicates with a 95% cut-off value. The specificity of the assay was assessed by purification and analysis of a series of DNA from whole blood of 24 healthy donors without detecting any methylated allele. The total failure rate of unmethylated allele PCR was 0%. Inhibitors were not observed due to the high efficiency of DNA extraction and bisulfite treated DNA purification.

## DETAILED DESCRIPTION OF THE INVENTION

The present invention provides a new, rapid method for promoter methylation analysis of MGMT gene. The present invention also provides a kit which could be particularly useful for a route clinical assay in the detection of general cancer and determination of special type of cancer concerning promoter hypermethylation of MGMT gene. Given the key roles of cytosine methylation, there has been a huge interest in the development of procedures for DNA methylation analyses[18]. Sensitive, accurate, high-throughput and cost-effective methods should give a better definition of the role and control of this epigenetic modification, especially in pathological situations such as cancer. Many different experimental approaches have been developed to allow either global, large-scale or specific analyses [2,3,6,18-26]. The most popular approaches rely on bisulfite treatment of DNA[41]. This treatment can be performed in such a way that, while cytosine is quantitatively deaminated to uracil, 5-methyl cytosine remains unmodified, thus allowing identification of the cytosine methylation status following PCR amplification. There are many possible variations in the subsequent DNA sequence detection methods used which achieve diverse levels of performance and adequacy to the analyses. The quantitative analysis of the cytosine methylation levels at several independent proximal positions can be achieved using Pyrosequencing analysis of PCR products amplified in a methylation-independent manner[21,41]. This procedure is not particularly sensitive and does not perform well in the case of the detection of a population that represents <5-10% of the total, which limits its usefulness in the analysis of heterogeneous pathological samples and early events in epigenetic reprogramming. On the other hand it gives information on each CpG present in the island of interest. Methylation-specific PCR (MS-PCR)[42] is the most widely used assay for the detection of hypermethylation in CpG islands. It relies on the selective PCR amplification of sequences corresponding to either unmethylated or methylated DNA using primers that anneal specifically with either one of the DNA species. For methylation-specific annealing, each primer must contain sequences corresponding to at least two CpG dinucleotides. Thus, MS-PCR can clearly identify the DNA molecules whose methylation statuses at multiple CpGs differ. This method allows sensitive detection of particular methylation patterns and appears very promising for the analysis of pathological samples. The MS-PCR approach has been adapted to fluorescence-based real-time PCR analyses, thus providing both a quantitative and high-throughput technology [20,21,28,29,42,43]. These approaches are thus best suited to the analysis of extensive changes in the methylation patterns within CpG islands, but they may provide some aspecific results due to aspecific annealing of primers especially after a great number of amplification cycles. Locked nucleic acid (LNA) based PCR to detect single nucleotide polymorphism (SNP) has been widely used to achieve higher specificity. LNA primers show very accurate mismatch discrimination in comparison with DNA primers at all 3' terminal positions[37]. Moreover it offers greater sensitivity respect to TaqMan based SNP detection due to the fact that in AS-PCR two PCR amplify selectively each single allele of the two. On the contrary, in case of TaqMan detection, a single PCR with flanking primers amplify in parallel both alleles. In case of a very low level of one allele of the two (mosaicism) due to the low density of tumor cells in a background of normal population, TaqMan detection method with discriminating probes is not the ideal one. This invention is related to a method detecting and quantifying even low-percentage mosaicism for methylated alleles against a background of unmethylated alleles by direct real-time quantitative PCR (qPCR) assay taking advantage of 3' locked nucleic acid (LNA) allele-specific PCR (AS-PCR) primers and by molecular beacon detection chemistry. One conceptually simple strategy to detect single-base substitutions is AS-PCR, which is based on positioning the 3' base of a PCR primer to match one variant allele[44]. Over the years, a number of strategies have been developed to improve the specificity and reliability of primers in AS-PCR. Among these, are the incorporation of additional mismatches near the 3' end[44] and the use of high-affinity DNA analogues such as LNAs [37,45]. Although AS-PCR assays provide an elegant method to discriminate between alleles, accurate quantification of the variants is not attainable because of the intrinsic endpoint detection by conventional PCR methods. This limitation is fully addressed using real-time qPCR methods[38], whereby PCR product accumulation is monitored at each PCR cycle by means of fluorescent detection using molecular beacon chemistry. Unlike previously described protocols[28-29], this new approach is based on two different detection beacon probes, one recognizing the methylated allele and the other one recognizing the unmethylated allele. We identify *SNURF* as the hypothetically ideal reference gene for its imprinted status and because it has been mapped at 15q12, a locus not previously reported for common aberrations in gliomas. The Ct difference between methylated and unmethylated allele was found to be very close to 1 Ct in the most part of cases. The aberrant methylation pattern found in a few cases might derive from a distinct CpG methylation pattern among tumor cell population, or from partially loss of one allele, or may be due to a methylation machinery disorder which might methylate the paternal allele. We use an imprinted gene as a reference, instead to use a methylation independent calibrator such as ACTB as indicated elsewhere[28], because ACTB has been mapped on 7p15-p12, and this locus was found to be deleted in some cases of gliomas grade I-III[47]. Following the guideline of relative quantifications by real time PCR, a reference gene should be stable, unaffected by experimental conditions and it should mimic the gene to be investigated. Actually, in healthy donors the maternal allele of SNURF is methylated at the promoter locus, while the paternal allele of the same gene is usually unmethylated and expressed. This condition is very similar to a given tumor population of cells in which the MGMT is methylated for one allele of the two. Using a 1.5 μg mixture of equal amount of SssI-treated DNA and untreated DNA as a calibrator, the MGMT MSP-qPCR helps us to verify parallel mMGMT and uMGMT amplification during each run. Moreover a dilution series of titrated bisulfite treated DNA from healthy female donors (Promega), was used to create a standard curve that was useful to calculate the amount of starting DNA for each unknown sample for uMGMT allele. In our hands the EpiTect Bisulfite kit allows to start from lower amount of FFPE derived DNA respect to more classic protocols[6] for its ability to reduce the intrinsic DNA fragmentation during the bisulfite treatment. Because the methylated and unmethylated control DNA for MGMT promoter yielded pure products only with mMGMT and uMGMT primers, we could show that the MS-qPCR assay can clearly discriminate between the methylated and unmethylated status of the MGMT promoter. Additionally, we could show that variations of standard curves used for calculations of MS-qPCR values in each run were small and acceptable, indicating a good reproducibility. Combining these highly accurate settings, the MS-qPCR technique was particularly suitable for detecting and quantify fully methylated and unmethylated DNA and it was able to display the correct proportions of methyalted and unmethylated DNA amounts with excellent precision starting from FFPE samples. Primers and probes were designed to test at least two or three CpGs for each primer and probe sequence, with respect to CpGs known to be methylated at high frequency in this kind of tumors[4]. By this assay several tumor samples can be investigated in one real-time PCR run in parallel, which is time saving and therefore clearly less expensive. Moreover by defining a relation between fully methylated and unmethylated portions of the MGMT promoter in terms of percentage, MS-qPCR provides more detailed information about the overall methylation status of the samples, and by this it offers the possibility to specify a more precise correlation with the temozolomide treatment.

### *In Vitro* Methylation Assay and Standards

To test for sensitivity and specificity of MS-qPCR, a titration experiments were performed using normal pooled genomic DNA (DNA Female pool, Cod. G1521, Promega, Milan) which was methylated *in vitro* using *SssI* (New England Biolabs, Milan). In brief, 1.5 μg was treated with *SssI* to methylate all CpG sites (near complete methylation and no loss of DNA was assumed) for two hours at 37°C following the instruction of the provider. Mixtures of *SssI*-treated DNA: untreated DNA (100%, 50%, 10%, 1%, 0.1% 0.01% ) were prepared in duplicate (each containing 1.5 μg of template DNA). 50% *SssI*-treated DNA: untreated DNA served as calibrator for MGMT MSP-qPCR, while the same DNA amount of untreated DNA pool (Cod. G1521 from Promega) served as calibrator for SNURF MSP-qPCR to confirm equal amount of maternal methylated allele and paternal unmethylated one. The same DNA pool was used to test for specificity in terms of absence of amplicons for the MGMT methylated allele for single run.

### MS-qPCR

Real Time PCR analysis (MS-qPCR) was performed using an SDS- ABI Prism 7000 (Applera). 3'-locked nucleic acid (LNA) primers (see Table 1) were synthesized by SIGMA-Proligo. Reactions were performed in a final volume of 25 μl, adjusted to 4 mM of $MgCl_2$ and containing 1 Unit of FastStartTaq DNA Polymerase (Roche, Milan), 1x related Buffer and 5 μl of 5x GC rich solution, 200 μM of dNTPs, 0.5 μl of ROX 50x (Invitrogen, Milan), 500 nM of each primers, 250 nM of beacon probe and 3 μl of bisulfite treated DNA. The real time qPCR cycling conditions were as follows: 95°C for 4 min, 60 °C for 2 min, 72 °C for 2 min, followed by 40 cycles for 20 s at 95 °C, 45 s at 60 °C with fluorescence measurement, 30 s at 72 °C.

### Relative Quantification of Methylated Allele

The number of PCR cycles (*Ct*) required for the FAM intensities to exceed a threshold just above background was calculated for the test (MGMT) and reference (SNURF) reactions: *Ct* values were determined for each sample and subtracted to obtain:

$$\Delta Ct\ [\Delta Ct = Ct\ [mMGMT - uMGMT] - Ct\ [(mSNURF - uSNURF)].$$

(mMGMT: methylated allele ; uMGMT: unmethylated allele; mSNURF: methylated allele; uSNURF: unmethylated allele)

ΔCt values were measured for each unknown sample [ΔCt (test DNA)] and for calibrator [ΔCt (equal amount of *Sss*I-treated DNA mixed with the same amount of untreated DNA)].

Relative copy number at each locus in the test sample was then calculated as:

Relative Methylated allele copy number = $(1+E)^{-\Delta\Delta Ct}$ where:

ΔΔCt = ΔCt (test DNA) - ΔCt (calibrator DNA), and $E$ = PCR efficiency. The efficiency of PCR was calculated from the slope of the line, E = 10 $^{-1/slope}$ - 1. For simplicity, we used primers with PCR efficiencies of >95% and we calculated the relative DNA copy number ratio respect the unmethylated allele following the formula = $2^{-\Delta\Delta Ct}$ as previously described.

A sample of 24 healthy controls was used to estimate the normality range of the differences between methylated and unmethylated SNURF alleles; the normality range was estimated as the mean value $\pm$ 1.96 * standard deviation.

**Primers and molecular beacon probes design**

Primers for methylated and unmethylated MGMT promoter were previously described[22,35] except for the insertion of LNA nucleotides at 3' (See Table 1). Primers for SNURF were designed using MethPrimer (http://www,urogene, org/methprimer/index1.html0)[36] and verified by methBlast (http://medgen,ugent.be/methBLAST/) (see Fig. 1 and Table 1).

Molecular beacon probes (see Table 1) were designed using the Primer3 software (http://frodo.wi.mit.deu/cgi-bin/primer3/primer3 www.cgi). Flanked molecular beacon arms were designed using the OLIGO 6.0 software reaching a temperature between 57 and 61°C in the stem loop conformation. LNA nucleotide were added to primers and beacon probes as described previously[37] using a Tm prediction tool available at www.exiqon.com. Amplicons were tested by MFOLD (http://www.bioinfo.rpi.edu/applications/mfold/old/dna/) in order to avoid secondary structures within primer and probe positions.

SEQ ID 1 MGMT MET-FOR TTT**C**GA**C**GTTCGTAGGTTTT**C**G+**C**
SEQ ID 2 MGMT MET-REV **G**CACTCTTCC**G**AAAAC**G**AAAC+**G**
SEQ ID 3 MGMT UMET-FOR TTTGTGTTTT**T**GA**T**GTTT**T**GTAGGTTTT**T**G+**T**
SEQ ID 4 MGMT UMET-REV AACTCC**A**CACTCTTCC**A**AAAAC**A**AAAC+**A**
SEQ ID 5 SNURF MET-FOR GGATTTTTGTATTG+**C**GGTAAATAAGTA+**C**
SEQ ID 6 SNURF MET-REV C**G**CTACAACAAC+**G**ACAAACTTC+**G**
SEQ ID 7 SNURF UMET-FOR GGATTTTTGTATTG+**T**GGTAAATAAGTA+**T**
SEQ ID 8 SNURF UMET-REV CTC**A**CTACAACAAC+**A**ACAAACTTC+**A**
SEQ ID 9 MGMT MET-BEACON FAM- CCGGAGCGTAT**C**GTTTG**C**GATTTGGTGAGTGTGCTCCGG-BHQ1
SEQ ID 10 MGMT UMET-BEACON FAM-CCGGTGCTGTAT**T**GTTTG**T**GATTTGGTGAGTGTGCACCGG-BHQ1
SEQ ID 11 SNURF MET-BEACON FAM- CCGGAGGGAGGTAGGTTGG**C**G**C**GTATGTTTAGCCTCCGG-BH1
SEQ ID 12 SNURF UMET-BEACON FAM- CCGGAGGGAGGTAGGTTGG**T**G**T**GTATGTTTAGGCCTCCGG-BH1

Related references

1. Laird PW. Cancer epigenetics. Hum Mol Genet 2005;14:R65-76.

2. Herman JG, Baylin SB. Gene silencing in cancer in association with promoter hypermethylation. N Engl J Med 2003;349:2042-2054.

3. Komine C, Watanabe T, Katayama Y, et al. Promoter hypermethylation of the DNA repair gene 06-methylguanine-DNA methyltransferase is an independent predictor of shortened progression free survival in patients with low-grade diffuse astrocytomas. Brain Pathol 2003;13:176-184.

4. Qian XC, Brent TP. Methylation hot spots in the 5' flanking region denote silencing of the 06-methylguanine-DNA methyltransferase gene. Cancer Res 1997;57:3672-3677.

5. Watts GS, Pieper RO, Costello JF, et al. Methylation of discrete regions of the O6-methylguanine DNA methyltransferase (MGMT) CpG island is associated with heterochromatinization of the MGMT transcription start site and silencing of the gene. Mol Cell Biol 1997;17:5612-5619.

6. Esteller M, Hamilton SR, Burger PC, et al. Inactivation of the DNA repair gene O6-methylguanine-DNA methyltransferase by promoter hypermethylation is a common event in primary human neoplasia. Cancer Res 1999;59: 793-797.

7. Toyota M, Ahuja N, Ohe-Toyota M, et al. CpG island methylator phenotype in colorectal cancer. Proc. Natl.Acad. Sci. USA 1999;96, 8681-8686.

8. Issa, J.P. CpG island methylator phenotype in cancer. Nat. Rev. Cancer 2004; 4: 988-993.

9. Liu L, Markowitz S, Gerson SL. Mismatch repair mutations override alkyltransferase in conferring resistance to temozolomide but not to 1,3-bis(2-chloroethyl) nitrosourea. Cancer Res 1996;56: 5375-5379.

10. Gerson SL. MGMT: its role in cancer aetiology and cancer therapeutics. Nat Rev Cancer 2004;4:296-307.

11. Hotta T, Saito Y, Fujita H, et al. O6-alkylguanine-DNA alkyltransferase activity of human malignant glioma and its clinical implications. J Neurooncol 1994;21:135-40.

12. Belanich M, Pastor M, Randall T, et al. Retrospective study of the correlation between the DNA repair protein alkyltransferase and survival of brain tumor patients treated with carmustine. Cancer Res 1996;56:783-788.

13. Jaeckle KA, Eyre HJ, Townsend JJ, et al. Correlation of tumor 06 methylguanine-DNA methyltransferase levels with survival of malignant astrocytoma patients treated with bis-chloroethylnitrosourea: a Southwest Oncology Group study. J Clin Oncol 1998;16:3310-3315.

14. Friedman HS, McLendon RE, Kerby T, et al. DNA mismatch repair and O6-alkylguanine-DNA alkyltransferase analysis and response to Temodal in newly diagnosed malignant glioma. J Clin Oncol 1998;16:3851-3857.

15. Komine C, Watanabe T, Katayama Y, et al. Promoter hypermethylation of the DNA repair gene 06-methylguanine-DNA methyltransferase is an independent predictor of shortened progression free survival in patients with low-grade diffuse astrocytomas. Brain Pathol 2003;13:176-184.

16. Stupp R, Mason WP, van den Bent MJ, et al. Radiotherapy plus concomitant and adjuvant temozolomide for glioblastoma. N Engl J Med 2005;352:987-996.

17. Hegi ME, Diserens AC, Gorlia T, et Al. MGMT gene silencing and benefit from temozolomide in glioblastoma N Engl J Med 2005;352:997-1003.

18. Kress C, Thomassin H, Grange T. Local DNA demethylation: how could it be achieved precisely, quickly and safely? FEBS Lett 2001;494:135-140.

19. Liu,Z.J. and Maekawa,M. Polymerase chain reaction-based methods of DNA methylation analysis. Anal. Biochem 2003;317:259-265.

20. Cottrell SE, Distler J, Goodman NS, et al. A real-time PCR assay for DNA-methylation using methylation-specific blockers. Nucleic Acids Res 2004; 32: E10.

21. Uhlmann K, Brinckmann A, Toliat MR, et al. Evaluation of a potential epigenetic biomarker by quantitative methyl-single nucleotide polymorphism analysis. Electrophoresis 2002; 23:4072-4079.

22. Esteller M, Garcia-Foncillas J, Andion E, et al. Inactivation of the DNA-repair gene MGMT and the clinical response of gliomas to alkylating agents. N Engl J Med 2000;343:1350-1354. [Erratum, N Engl J Med 2000;343:1740.]

23. Jeuken JW, Cornelissen SJ, Vriezen M, et al. MS-MLPA: an attractive alternative laboratory assay for robust, reliable, and semiquantitative detection of MGMT promoter hypermethylation in gliomas. Lab Investigation 2007; 87:1055-1065

24. Cankovic M, Mikkelsen T, Rosenblum ML, et al. A simplified laboratory validated assay for MGMT promoter hypermethylation analysis of glioma specimens from formalin-fixed paraffin-embedded tissue. Lab Invest. 2007; 87: 392-397.

25. Thomassin H, Oakeley EJ, Grange T. (1999) Identification of 5-methylcytosine in complex genomes. Methods 1999;19:465-475.

26. Fraga MF, Esteller M. DNA methylation: a profile of methods and applications. Biotechniques 2002;33:632-649.

27. Eads CA, Danenberg KD, Kawakami K, et al. MethyLight: a high-throughput assay to measure DNA methylation. Nucleic Acids Res 2000;28:E32.

28. Vlassenbroeck I, Califice S, Diserens AC, et al. Validation of real-time methylation-specific PCR to determine 06-methylguanine-DNA methyltransferase gene promoter methylation in glioma. J Mol Diagn. 2008;10:332-337.

29. Wojdacz TK, Dobrovic A Methylation-sensitive high resolution melting (MS-HRM): a new approach for sensitive and high-throughput assessment of methylation. Nucleic Acids Res. 2007;35:e41.

30. El-Maarri O, Buiting K, Peery EG, et al. Maternal methylation imprints on human chromosome 15 are established during or after fertilization. Nat Genet. 2001;27:341-344.

31. Ginzinger DG, Godfrey TE, Nigro J, et al. Measurement of DNA copy number at microsatellite loci using quantitative PCR analysis. Cancer Res. 2000;60:5405-5409.

32. Louis DN, Ohgaki H, Wiestler OD, et al. World Health Organization Classification of tumours of the central nervous system. IARC, Lyon 2007

33. Morandi L, Marucci G, Foschini MP, et al. Genetic similarities and differences between lobular in situ neoplasia (LN) and invasive lobular carcinoma of the breast. Virchows Arch. 2006;449:14-23.

34. Palmisano WA, Divine KK, Saccomanno G, et al. Predicting lung cancer by detecting aberrant promoter methylation in sputum. Cancer Res 2000;60:5954-5958.

35. van Engeland M, Weijenberg MP, Roemen GM, et al. Effects of Dietary Folate and Alcohol Intake on Promoter Methylation in Sporadic Colorectal Cancer: The Netherlands Cohort Study on Diet and Cancer. Cancer Research 2003;63:3133-3137.

36. Li LC, Dahiya R. MethPrimer: designing primers for methylation PCRs. Bioinformatics 2002;18:1427-1431

37. D. Latorra, K. Campbell, A. Wolter, et al. Enhanced allele-specific PCR discrimination in SNP genotyping using 3' locked nucleic acid (LNA) primers, Hum. Mutat 2003;22:79-85.

38. Maertens O, Legius E, Speleman F, et al. Real-time quantitative allele discrimination assay using 3' locked nucleic acid primers for detection of low-percentage mosaic mutations. Anal Biochem. 2006;359:144-146.

39. Liikanen E. Common technical specification for in vitro diagnostic medical devices. Official Journal of European Community. 2002;L131:17-30

40. Frommer M, McDonald LE, Millar DS, et al. A genomic sequencing protocol that yields a positive display of 5-methylcytosine residues in individual DNA strands. Proc. Natl Acad. Sci. USA 1992;89:1827-1831.

41. Tost J, Dunker J,Gut IG Analysis and quantification of multiple methylation variable positions in CpG islands by Pyrosequencing. Biotechniques 2003;35:152-156.

42. Herman JG, Graff JR, Myohanen S, et al. Methylation-specific PCR: a novel PCR assay for methylation status of CpG islands. Proc. Natl Acad. Sci. USA 1996;93:9821-9826.

43. Thomassin H, Kress C, Grange T. MethylQuant: a sensitive method for quantifying methylation of specific cytosines within the genome. Nucleic Acids Research 2004;32:E 21

44. Bottema CD, Sommer SS. PCR amplification of specific alleles: rapid detection of known mutations and polymorphisms. Mutat Res 1993;288:93-102.

45. Newton CR, Graham A, Heptinstall LE, Powell SJ, Summers C, Kalsheker N, Smith JC, Markham AF. Analysis of any point mutation in DNA: the amplification refractory mutation system (ARMS), Nucleic Acids Res 1989;17:2503-2516.

46. Latorra D, Arar K, Hurley JM. Design considerations and effects of LNA in PCR primers, Mol. Cell. Probes 2003; 17:253-259.

47. Roversi G, Pfundt R, Moroni RF, et al. Identification of novel genomic markers related to progression to glioblastoma through genomic profiling of 25 primary glioma cell lines. Oncogene. 2006;25:1571-1583.

48. Ohgaki H, Kleihues P. Genetic pathways to primary and secondary glioblastoma. Am J Pathol. 2007;170:1445-1453

49. Preusser M, Charles Janzer R, Felsberg J, Reifenberger G, Hamou MF, Diserens AC, Stupp R, Gorlia T, Marosi C, Heinzl H, Hainfellner JA, Hegi M. Anti-O6-methylguanine-methyltransferase (MGMT) immunohistochemistry in glioblastoma multiforme: observer variability and lack of association with patient survival impede its use as clinical biomarker. Brain Pathol. 2008;18:520-532

50. Curtin NJ, Wang LZ, Yiakouvaki A, et al. Novel poly(ADP-ribose) polymerase-1 inhibitor, AG14361, restores sensitivity to temozolomide in mismatch repair-deficient cells. Clin Cancer Res 2004;10:881-889.

51. Trivedi RN, Almeida KH, Fornsaglio JL, et al. The role of base excision repair in the sensitivity and resistance to temozolomide-mediated cell death. Cancer Res 2005;65:6394-6400.

**Claims**

1. A method for quantifying the methylated allele of 06-methylguanine-DNA methyltransferase (MGMT) promoter DNA in cancer cells, comprising: assaying for quantifying the methylated allele of MGMT promoter DNA in formalin fixed paraffin embedded (FFPE) by methylation sensitive quantitative PCR.

2. The method of claim 1, wherein the specimen is fresh tissue

3. The method of claim 1, wherein the specimen is frozen tissue

4. The method of claim 1, wherein the specimen is tissue fixed by ethanol based fixatives.

5. The method of claim 1, wherein the tumor DNA is isolated and unmethylated cytosines are converted into uracil by bisulfite treatment

6. The method of claim 1, wherein a reference gene is an imprinted gene which has by definition one hypermethylated allele and one unmethylated allele.

7. The method of claim 1, wherein a reference gene is SNURF, mapped on 15q12, a stable chromosomal region in many tumor types including gliomas

8. The method of claim 1, wherein said quantitative PCR amplification uses a group of primers as indicated in SEQ ID No1,2,3,4,5,6,7,8

9. The method of claim 1, wherein said PCR amplification uses a group of molecular beacon probe as indicated in SEQ ID No 9,10,11,12

10. The method of claim 1, wherein the method of calculating the ratio between methylated allele/unmethylated allele follows the indications described by Ginzinger DG, Godfrey TE, Nigro J, et al . (Measurement of DNA copy number at microsatellite loci using quantitative PCR analysis. Cancer Res. 2000;**60**:5405-5409) with some modifications. The number of PCR cycles (Ct) required for the FAM intensities to exceed a threshold just above background is calculated for the test (MGMT) and reference (SNURF) reactions: Ct values were determined for each sample and subtracted to obtain: ΔCt [ΔCt = Ct [mMGMT - uMGMT] - Ct [(mSNURF - uSNURF)]. (mMGMT: methylated allele ; uMGMT: unmethylated allele; mSNURF: methylated allele; uSNURF: unmethylated allele). ΔCt values were measured for each unknown sample [ΔCt (test DNA)] and for calibrator [ΔCt (equal amount of *Sss*I-treated DNA mixed with the same amount of untreated DNA)]. Relative copy number at each locus in the test sample was then calculated as: Relative Methylated allele copy number = $(1+E)^{-\Delta\Delta Ct}$ where: $\Delta\Delta Ct$ = ACt (test DNA) - ΔCt (calibrator DNA), and $E$ = PCR efficiency. The efficiency of PCR was calculated from the slope of the line, $E = 10^{-1/slope} - 1$. For simplicity, we used primers with PCR efficiencies of >95% and we calculated the relative DNA copy number ratio respect the unmethylated allele following the formula = $2^{-\Delta\Delta Ct}$ as previously described (Ginzinger et Al.).

11. The method of claim 1, wherein said a kit is comprised of a FastDNAFFPE solution for quick DNA extraction from FFPE tissue samples, comprising various salt, detergents and a protein-degrading enzyme, a kit for bisulfite treatment, a methylation/demethylation-specific DNA amplification system comprising a set of primer pairs, a set of oligonucleotide probes labeled with different dyes, DNA polymerase and amplification reaction buffer; and an instruction for conducting an assay according to the method of this invention.

# EP 2 218 793 A1

Europäisches Patentamt
European Patent Office
Office européen des brevets

## EUROPEAN SEARCH REPORT

Application Number

EP 10 15 3536

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 900 825 A1 (UNIV BONN [DE]) 19 March 2008 (2008-03-19) * page 2 - page 20 * ----- | 1-11 | INV. C12Q1/68 |
| X | WO 2005/089414 A2 (UNIV JOHNS HOPKINS [US]; SIDRANSKY DAVID [US]) 29 September 2005 (2005-09-29) * page 2 - page 40 * ----- | 1-11 | |
| X | WO 02/18649 A1 (LOVELACE RESPIRATORY RES INST [US]; BELINSKY STEVEN A [US]; PALMISANO) 7 March 2002 (2002-03-07) * page 1 - page 30 * ----- | 1-11 | |
| A | WO 2008/154590 A2 (MAYO FOUNDATION [US]; SARKARIA JANN N [US]; KITANGE GASPER J [US]; CAR) 18 December 2008 (2008-12-18) * page 3 - page 56 * ----- | 1-11 | |
| A | PELLOSKI C E ET AL: "MGMT Promoter Methylation is an Independent Prognostic Factor in the Absence of Alkylating Chemotherapy in Glioblastoma" INTERNATIONAL JOURNAL OF RADIATION: ONCOLOGY BIOLOGY PHYSICS, PERGAMON PRESS, USA LNKD-DOI:10.1016/J.IJROBP.2008.06.787, vol. 72, no. 1, 1 September 2008 (2008-09-01), page S9, XP025642663 ISSN: 0360-3016 [retrieved on 2008-09-01] * page 46 - page 55 * ----- -/-- | 1-11 | TECHNICAL FIELDS SEARCHED (IPC) C12Q |

The present search report has been drawn up for all claims

| Place of search | Date of completion of the search | Examiner |
|---|---|---|
| Munich | 30 April 2010 | Brochado Garganta, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

9

Europäisches
Patentamt

European
Patent Office

Office européen
des brevets

**EUROPEAN SEARCH REPORT**

Application Number

EP 10 15 3536

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HASSLER M ET AL: "Diversity of cytogenetic and pathohistologic profiles in glioblastoma" CANCER GENETICS AND CYTOGENETICS, ELSEVIER SCIENCE PUBLISHING, NEW YORK, NY, US LNKD-DOI:10.1016/J.CANCERGENCYTO.2005.08.021, vol. 166, no. 1, 1 April 2006 (2006-04-01), pages 46-55, XP024999490 ISSN: 0165-4608 [retrieved on 2006-04-01] * the whole document * | 1-11 | |
| A | COHEN ET AL: "Hypermethylation of CpG island loci of multiple tumor suppressor genes in retinoblastoma" EXPERIMENTAL EYE RESEARCH, ACADEMIC PRESS LTD, LONDON LNKD-DOI:10.1016/J.EXER.2007.10.010, vol. 86, no. 2, 4 November 2007 (2007-11-04), pages 201-206, XP022486578 ISSN: 0014-4835 * the whole document * | 1-11 | TECHNICAL FIELDS SEARCHED (IPC) |

The present search report has been drawn up for all claims

| Place of search | Date of completion of the search | Examiner |
|---|---|---|
| Munich | 30 April 2010 | Brochado Garganta, M |

EPO FORM 1503 03.82 (P04C01)

**ANNEX TO THE EUROPEAN SEARCH REPORT
ON EUROPEAN PATENT APPLICATION NO.**                    EP 10 15 3536

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2010

| Patent document cited in search report | | Publication date | Patent family member(s) | | Publication date |
|---|---|---|---|---|---|
| EP 1900825 | A1 | 19-03-2008 | NONE | | |
| WO 2005089414 | A2 | 29-09-2005 | CA | 2558649 A1 | 29-09-2005 |
| | | | EP | 1730160 A2 | 13-12-2006 |
| | | | US | 2009023134 A1 | 22-01-2009 |
| WO 0218649 | A1 | 07-03-2002 | AT | 347619 T | 15-12-2006 |
| | | | AU | 8837901 A | 13-03-2002 |
| | | | CA | 2421102 A1 | 07-03-2002 |
| | | | DE | 60125065 T2 | 28-06-2007 |
| | | | EP | 1352089 A1 | 15-10-2003 |
| WO 2008154590 | A2 | 18-12-2008 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Herman et al.** *Proc. Natl. Acad. Sci. USA,* 1996, vol. 93, 9821-9826
- **Eads et al.** *Cancer Res,* 1999, vol. 59, 2302-2306
- **Cottrell et al.** *Nucleic Acid Res,* 2004, vol. 32, e10
- **Laird PW.** Cancer epigenetics. *Hum Mol Genet,* 2005, vol. 14, R65-76
- **Herman JG ; Baylin SB.** Gene silencing in cancer in association with promoter hypermethylation. *N Engl J Med,* 2003, vol. 349, 2042-2054
- **Komine C ; Watanabe T ; Katayama Y et al.** Promoter hypermethylation of the DNA repair gene 06-methylguanine-DNA methyltransferase is an independent predictor of shortened progression free survival in patients with low-grade diffuse astrocytomas. *Brain Pathol,* 2003, vol. 13, 176-184
- **Qian XC ; Brent TP.** Methylation hot spots in the 5' flanking region denote silencing of the 06-methylguanine-DNA methyltransferase gene. *Cancer Res,* 1997, vol. 57, 3672-3677
- **Watts GS ; Pieper RO ; Costello JF et al.** Methylation of discrete regions of the O6-methylguanine DNA methyltransferase (MGMT) CpG island is associated with heterochromatinization of the MGMT transcription start site and silencing of the gene. *Mol Cell Biol,* 1997, vol. 17, 5612-5619
- **Esteller M ; Hamilton SR ; Burger PC et al.** Inactivation of the DNA repair gene O6-methylguanine-DNA methyltransferase by promoter hypermethylation is a common event in primary human neoplasia. *Cancer Res,* 1999, vol. 59, 793-797
- **Toyota M ; Ahuja N ; Ohe-Toyota M et al.** CpG island methylator phenotype in colorectal cancer. *Proc. Natl.Acad. Sci. USA,* 1999, vol. 96, 8681-8686
- **Issa, J.P.** CpG island methylator phenotype in cancer. *Nat. Rev. Cancer,* 2004, vol. 4, 988-993
- **Liu L ; Markowitz S ; Gerson SL.** Mismatch repair mutations override alkyltransferase in conferring resistance to temozolomide but not to 1,3-bis(2-chloroethyl) nitrosourea. *Cancer Res,* 1996, vol. 56, 5375-5379
- **Gerson SL.** MGMT: its role in cancer aetiology and cancer therapeutics. *Nat Rev Cancer,* 2004, vol. 4, 296-307
- **Hotta T ; Saito Y ; Fujita H et al.** O6-alkylguanine-DNA alkyltransferase activity of human malignant glioma and its clinical implications. *J Neurooncol,* 1994, vol. 21, 135-40

- **Belanich M ; Pastor M ; Randall T et al.** Retrospective study of the correlation between the DNA repair protein alkyltransferase and survival of brain tumor patients treated with carmustine. *Cancer Res,* 1996, vol. 56, 783-788
- **Jaeckle KA ; Eyre HJ ; Townsend JJ et al.** Correlation of tumor 06 methylguanine-DNA methyltransferase levels with survival of malignant astrocytoma patients treated with bis-chloroethylnitrosourea: a Southwest Oncology Group study. *J Clin Oncol,* 1998, vol. 16, 3310-3315
- **Friedman HS ; McLendon RE ; Kerby T et al.** DNA mismatch repair and O6-alkylguanine-DNA alkyltransferase analysis and response to Temodal in newly diagnosed malignant glioma. *J Clin Oncol,* 1998, vol. 16, 3851-3857
- **Stupp R ; Mason WP ; van den Bent MJ et al.** Radiotherapy plus concomitant and adjuvant temozolomide for glioblastoma. *N Engl J Med,* 2005, vol. 352, 987-996
- **Hegi ME ; Diserens AC ; Gorlia T et al.** MGMT gene silencing and benefit from temozolomide in glioblastoma. *N Engl J Med,* 2005, vol. 352, 997-1003
- **Kress C ; Thomassin H ; Grange T.** Local DNA demethylation: how could it be achieved precisely, quickly and safely?. *FEBS Lett,* vol. 494, 135-140
- **Liu,Z.J. ; Maekawa,M.** Polymerase chain reaction-based methods of DNA methylation analysis. *Anal. Biochem,* 2003, vol. 317, 259-265
- **Cottrell SE ; Distler J ; Goodman NS et al.** A real-time PCR assay for DNA-methylation using methylation-specific blockers. *Nucleic Acids Res,* 2004, vol. 32, E10
- **Uhlmann K ; Brinckmann A ; Toliat MR et al.** Evaluation of a potential epigenetic biomarker by quantitative methyl-single nucleotide polymorphism analysis. *Electrophoresis,* 2002, vol. 23, 4072-4079
- **Esteller M ; Garcia-Foncillas J ; Andion E et al.** Inactivation of the DNA-repair gene MGMT and the clinical response of gliomas to alkylating agents. *N Engl J Med,* 2000, vol. 343, 1350-1354
- *N Engl J Med,* 2000, vol. 343, 1740
- **Jeuken JW ; Cornelissen SJ ; Vriezen M et al.** MS-MLPA: an attractive alternative laboratory assay for robust, reliable, and semiquantitative detection of MGMT promoter hypermethylation in gliomas. *Lab Investigation,* 2007, vol. 87, 1055-1065

- **Cankovic M ; Mikkelsen T ; Rosenblum ML et al.** A simplified laboratory validated assay for MGMT promoter hypermethylation analysis of glioma specimens from formalin-fixed paraffin-embedded tissue. *Lab Invest.,* 2007, vol. 87, 392-397
- **Thomassin H ; Oakeley EJ ; Grange T.** Identification of 5-methylcytosine in complex genomes. *Methods,* 1999, vol. 19, 465-475
- **Fraga MF ; Esteller M.** DNA methylation: a profile of methods and applications. *Biotechniques,* 2002, vol. 33, 632-649
- **Eads CA ; Danenberg KD ; Kawakami K et al.** MethyLight: a high-throughput assay to measure DNA methylation. *Nucleic Acids Res,* 2000, vol. 28, E32
- **Vlassenbroeck I ; Califice S ; Diserens AC et al.** Validation of real-time methylation-specific PCR to determine 06-methylguanine-DNA methyltransferase gene promoter methylation in glioma. *J Mol Diagn,* 2008, vol. 10, 332-337
- **Wojdacz TK ; Dobrovic A.** Methylation-sensitive high resolution melting (MS-HRM): a new approach for sensitive and high-throughput assessment of methylation. *Nucleic Acids Res.,* 2007, vol. 35, e41
- **El-Maarri O ; Buiting K ; Peery EG et al.** Maternal methylation imprints on human chromosome 15 are established during or after fertilization. *Nat Genet.,* 2001, vol. 27, 341-344
- **Ginzinger DG ; Godfrey TE ; Nigro J et al.** Measurement of DNA copy number at microsatellite loci using quantitative PCR analysis. *Cancer Res.,* 2000, vol. 60, 5405-5409
- **Louis DN ; Ohgaki H ; Wiestler OD et al.** World Health Organization Classification of tumours of the central nervous system. *IARC,* 2007
- **Morandi L ; Marucci G ; Foschini MP et al.** Genetic similarities and differences between lobular in situ neoplasia (LN) and invasive lobular carcinoma of the breast. *Virchows Arch,* 2006, vol. 449, 14-23
- **Palmisano WA ; Divine KK ; Saccomanno G et al.** Predicting lung cancer by detecting aberrant promoter methylation in sputum. *Cancer Res,* 2000, vol. 60, 5954-5958
- **van Engeland M ; Weijenberg MP ; Roemen GM et al.** Effects of Dietary Folate and Alcohol Intake on Promoter Methylation in Sporadic Colorectal Cancer: The Netherlands Cohort Study on Diet and Cancer. *Cancer Research,* 2003, vol. 63, 3133-3137
- **Li LC ; Dahiya R.** MethPrimer: designing primers for methylation PCRs. *Bioinformatics,* 2002, vol. 18, 1427-1431
- **D. Latorra ; K. Campbell ; A. Wolter et al.** Enhanced allele-specific PCR discrimination in SNP genotyping using 3' locked nucleic acid (LNA) primers. *Hum. Mutat,* 2003, vol. 22, 79-85
- **Maertens O ; Legius E ; Speleman F et al.** Real-time quantitative allele discrimination assay using 3' locked nucleic acid primers for detection of low-percentage mosaic mutations. *Anal Biochem.,* 2006, vol. 359, 144-146
- **Liikanen E.** Common technical specification for in vitro diagnostic medical devices. *Official Journal of European Community,* 2002, vol. L131, 17-30
- **Frommer M ; McDonald LE ; Millar DS et al.** genomic sequencing protocol that yields a positive display of 5-methylcytosine residues in individual DNA strands. *Proc. Natl Acad. Sci. USA,* 1992, vol. 89, 1827-1831
- **Tost J ; Dunker J.** Gut IG Analysis and quantification of multiple methylation variable positions in CpG islands by Pyrosequencing. *Biotechniques,* 2003, vol. 35, 152-156
- **Herman JG ; Graff JR ; Myohanen S et al.** Methylation-specific PCR: a novel PCR assay for methylation status of CpG islands. *Proc. Natl Acad. Sci. USA,* 1996, vol. 93, 9821-9826
- **Thomassin H ; Kress C ; Grange T.** MethylQuant: a sensitive method for quantifying methylation of specific cytosines within the genome. *Nucleic Acids Research,* 2004, vol. 32, E 21
- **Bottema CD ; Sommer SS.** PCR amplification of specific alleles: rapid detection of known mutations and polymorphisms. *Mutat Res,* 1993, vol. 288, 93-102
- **Newton CR ; Graham A ; Heptinstall LE ; Powell SJ ; Summers C ; Kalsheker N ; Smith JC ; Markham AF.** Analysis of any point mutation in DNA: the amplification refractory mutation system (ARMS). *Nucleic Acids Res,* 1989, vol. 17, 2503-2516
- **Latorra D ; Arar K ; Hurley JM.** Design considerations and effects of LNA in PCR primers. *Mol. Cell. Probes,* 2003, vol. 17, 253-259
- **Roversi G ; Pfundt R ; Moroni RF et al.** Identification of novel genomic markers related to progression to glioblastoma through genomic profiling of 25 primary glioma cell lines. *Oncogene,* 2006, vol. 25, 1571-1583
- **Ohgaki H ; Kleihues P.** Genetic pathways to primary and secondary glioblastoma. *Am J Pathol.,* 2007, vol. 170, 1445-1453
- **Preusser M ; Charles Janzer R ; Felsberg J ; Reifenberger G ; Hamou MF ; Diserens AC ; Stupp R ; Gorlia T ; Marosi C ; Heinzl H.** Anti-O6-methylguanine-methyltransferase (MGMT) immunohistochemistry in glioblastoma multiforme: observer variability and lack of association with patient survival impede its use as clinical biomarker. *Brain Pathol.,* 2008, vol. 18, 520-532
- **Curtin NJ ; Wang LZ ; Yiakouvaki A et al.** Novel poly(ADP-ribose) polymerase-1 inhibitor, AG14361, restores sensitivity to temozolomide in mismatch repair-deficient cells. *Cancer Res,* 2004, vol. 10, 881-889

• **Trivedi RN ; Almeida KH ; Fornsaglio JL et al.** The role of base excision repair in the sensitivity and resistance to temozolomide-mediated cell death. *Cancer Res,* 2005, vol. 65, 6394-6400